# FASCICULE DE BREVET EUROPEEN

(11) **EP 0 862 433 B1**
(45) Date de publication et mention de la délivrance du brevet: **11.09.2002**
(21) Numéro de dépôt: 96938270.4
(22) Date de dépôt: 07.11.1996
(51) Int. Cl.: A61K 31/44, A61K 9/12, A61P 31/10

(54) **COMPOSITION PHARMACEUTIQUE TOPIQUE MOUSSANTE POUR LE TRAITEMENT DES DERMATOSES, INDUITES PAR PITYROSPORUM OVALE**
TOPISCH ANZUWENDENDES ABSCHÄUMENDES ARZNEIMITTEL ZUR BEHANDLUNG VON DURCH PITYROSPORUM OVALE VERURSACHTEN HAUTERKRANKUNGEN
TOPICAL FOAMABLE PHARMACEUTICAL COMPOSITION FOR TREATING SKIN DISEASES INDUCED BY OVAL PITYROSPORUM

(30) Priorité: 08.11.1995 FR 9513202
(43) Date de publication de la demande: 09.09.1998
(73) Titulaire: PIERRE FABRE MEDICAMENT, 92100 Boulogne-Billancourt (FR)
(72) Inventeur: LEVERD, Elie, F-81100 Castres (FR); LACROUX, Martine, F-31750 Esacalquens (FR); MROZ-COMBESSIS, Danielle, F-31520 Ramonville-Saint-Agne (FR); BOUGARET, Jo[l, F-31570 Lanta (FR); MROZ, Christian, F-31520 Ramonville-Saint-Agne (FR)
(74) Mandataire: Warcoin, Jacques
(86) Numéro de dépôt international: FR9601753
(87) Numéro de publication internationale: WO97017075

(56) Documents cités:
- EP-A- 0 510 561
- FR-A- 2 191 904
- CHEMICAL ABSTRACTS, vol. 100, no. 18, 30 Avril 1984 Columbus, Ohio, US; abstract no. 144826, XP002008603 & JP 59 007 109 A (LION CORP.,JP) 14 Janvier 1984

## Description

La présente invention a pour objet des compositions pharmaceutiques moussantes à usage topique destinées au traitement des dermatoses induites par Pityrosporum ovale.

Elle concerne des solutions ou des gels moussants, à base de principe actif anti-fongique de structure issue de la pyridone tel que la ciclopiroxolamine.

Les agents antifongiques utilisés à ce jour pour le traitement de ces affections comprennent des antibiotiques de type macrolides ou autre (griséofulvine), des dérivés azolés, des composés soufres, des acides gras, etc.

En thérapeutique, le traitement local reste très important et concerne à lui seul une majorité de mycoses cutanée-muqueuses pour lesquelles les dérivés azolés sont les plus souvent employés qu'il S'agisse d'imidazoles (exemple le kétoconazole) ou de triazoles (exemple : le fluconazole).

Les auteurs s'intéressent plus particulièrement aux molécules à structure dérivée du ciclopirox ou 6-cyclohexyl-1-hydroxy-4-méthyl-2-(IH)-pyridinone sous forme de sels tels que la ciclopiroxolamine.

Ces molécules sont des anti-fongiques puissants et possèdent également des propriétés anti-bactériennes. L'existence d'un spectre large leur permet d'être indiquées dans le traitement des dermatophytoses à trichophyton, épidermophyton, microsporum (en dehors des teignes) des candidoses cutanées et du Pityriasis versicolor.

La présente invention ne concerne pas l'octopirox = piroctone = 1-hydroxy-4-méthyl-6-(2,4,4-triméthylpentyl)-2(1H)-pyridinone ou ses sels actifs qui sont utilisés généralement dans des préparations cosmétiques en tant qu'agents antiséborrhéiques ou anti-pelliculaires.

L'invention ne concerne que des préparations à base de sel de ciclopirox dans le cadre des pathologies dont le Pityrosporum ovale est le principal agent étiologique.

Ce champignon est encore connu sous le nom de : Malassezia furfur, Pityrosporum orbiculare, Microsporon furfur...

Dans un cadre général, le traitement d'une affection fongique cutanée n'est réellement efficace que si la spécialité pharmaceutique utilisée présente deux caractéristiques :
- une forme galénique appropriée, ce qui est le cas dans le traitement du pityriasis versicolor ou de la dermite séborrhéique avec une solution ou un gel moussant qui permettent, par leurs qualités dispersantes, de traiter localement la zone de surface corporelle ou du cuir chevelu.
- le respect de l'activité anti-fongique du principe-actif : en effet, comme le montre le présent texte, de nombreux tensio-actifs inhibent l'activité des dérivés du ciclopirox.

L'objectif général lors de la formulation de préparations moussantes est d'obtenir une mousse de bonne qualité, stable dans le temps. Pour se faire l'homme de l'art utilisera classiquement :
- un tensio-actif principal permettant de disperser le principe-actif, de créer la mousse, et de nettoyer la surface sur laquelle il est appliqué,
- un tensio-actif secondaire (ou booster de mousse) qui augmente les propriétés moussantes et stabilise la mousse.

Les tensio-actifs anioniques (type alkyl sulfates et/ou alkyl éther sulfates) sont généralement utilisés comme tensio-actif principal car ils ont de bonnes propriétés moussantes.

Les tensio-actifs secondaires sont généralement des tensio-actifs anioniques ou des amphotères.

Les non ioniques sont parfois ajoutés pour améliorer les caractéristiques de la mousse, ou comme solubilisant de parfum et/ou autres matériaux hydrophobes.

Les tensio-actifs cationiques sont peu utilisés pour les produits moussants, ils sont souvent réservés aux "après-shampooings" (comme "conditionneurs"),

Aussi les principaux surfactifs utilisés appartiennent notamment aux classes suivantes (seuls ou en mélange) :
- monoglycérides sulfates,
- alkyl sulfonates,
- monoalkyl sulfosuccinates,
auxquels on ajoute des tensio-actifs secondaires ou"boosters de mousse" :
- amines oxydes,
- bétaïnes¹.

Des épaississants tels que sels minéraux, silicate de magnésium ou d'aluminium; carboxyéthyl cellulose, carboxyméthyl cellulose,..., peuvent être ajoutés pour stabiliser la préparation dans le temps. Parfois des humectants sont également employés pour diminuer l'irritation liée à l'emploi de tensio-actifs notamment les tensio-actifs anioniques.
¹ P ALEXANDER Spotlight on shampoos - Manufacturing chemist 61, 11,39-43 (Nov.1990)

De nombreux brevets concernent des associations de tensio-actifs ayant pour but l'amélioration :
- du pouvoir nettoyant (EP-A-0075994 THE PROCTER & GAMBLE ; EP-A-010556 THE PROCTER & GAMBLE)
- ou l'amélioration des qualités moussantes (EP-A-0070076 THE PROCTER & GAMBLE) de préparations aqueuses.

Ce n'est pas le but de notre invention, dont les propriétés moussantes sont classiquement dues aux propriétés des tensio-actifs utilisés.

D'autres brevets concernent des associations de tensio-actifs faiblement irritants pour la peau (type tensio-actif glycosidique + tensio-actif amphotère + booster de mousse) qui permettent d'éviter l'emploi d'humectant ou d'autres additifs dans des préparations cosmétiques type savon liquide pour les mains ou bain moussant (brevet US 4,668,422 A.E.STALEY MANUFACTURING).

Le brevet (EP-A-0422 508 KAO CORPORATION) décrit des associations de tensioactifs saccharidiques non ioniques avec divers agents antibactériens dont le but est d'améliorer l'efficacité de ces actifs type antipelliculaires dans des shampooings. Les auteurs expliquent que le type de formulation utilisée permet grâce aux tensioactifs saccharidiques utilisés (octyl glucoside par exemple) de respecter les défenses naturelles du cuir chevelu face aux stimuli et donc d'améliorer notablement l'effet anti-bactérien des compositions.

Notre invention ne comporte pas de tensio-actif saccharidique ou glycosidique.

D'autres brevets décrivent une potentialisation de l'activité de principe actif antifongique soit :
- par une augmentation de la pénétration tissulaire de l'actif (81IL-064208) liée à l'utilisation d'un "enhancer",
- soit par une augmentation du temps de rétention de l'agent antipelliculaire au niveau des cheveux et du cuir chevelu :
   - EP-A-0347 199 (UNILEVER PLC) concerne un shampooing aqueux ayant d'excellentes propriétés moussantes à base de dialkylsulfosuccinate auquel est associé un agent anti-microbien choisi parmi les dérivés de: 1-chlorophénoxy, 1-imidazolyl-2-butanone, 1-hydroxy-2-pyridone;
   - EP-A-117 135 (JOHNSON & JOHNSON) décrit une composition détergente comportant un polymère hydrosoluble azoté + au moins un tensio-actif anionique ou amphotère + au moins un agent antipelliculaire non particulaire, soluble dans l'eau choisi parmi les 1-hydroxy-2-pyridone et les produits d'addition avec le sulfate de magnésium de 2,2'-dithio-bis-(1-oxyde de pyridine). Selon les auteurs la rétention au niveau capillaire est augmentée par la formation d'un complexe (coacervat) polymère hydrosoluble azoté/ tensio-actif.

EP-A-5 105 561 décrit des compositions pharmaceutiques moussantes contenant de la ciclopiroxolamine et des tensio-actifs tels que des alkylamidobétaïnes, mais toujours en combinaison avec d'autres agents servant a solubiliser la ciclopiroxolamine.
- Brevet US n° 4,835,148 (THE PROCTER & GAMBLE Co) concerne des shampooings à visée anti-inflammatoire comportant un principe actif insoluble dans l'eau, en suspension dans un véhicule aqueux (acétate d'hydrocortisone, pyridinethione sels, 1-hydroxy-2-pyridone) + des tensio-actifs anioniques (alkylsulfate ou alkyléther sulfate) + des agents de suspension.

Les brevets américains U.S. n° 4,711,775 (HOECHST AKTIENGESELLSCHAFT) et français n° 73 25464 (FARBWERKE HOECHST AKTIENGESELLSCHAFT VORMALS MEISTER LUCIUS & BRUNING) décrivent des compositions cosmétiques antipelliculaires contenant des dérivés de la 1-hydroxy-2-pyridone associés à divers tensio-actifs dans des produits capillaires, destinées à être appliquées un temps plus ou moins long sur la chevelure. Ces brevets ne revendiquent que des préparations cosmétiques et non des préparations pharmaceutiques.

L'objet de la présente invention n'est pas d'augmenter la rémanence des actifs au niveau de la peau ou du cuir chevelu. Par ailleurs, cette invention ne concerne ni les polymères hydrosolubles azotés ni les dialkylsulfosuccinates.

Dans le cadre de l'invention, il est nécessaire de solubiliser les dérivés de la pyridone utilisés pour réaliser les compositions moussantes pharmaceutiques à usage topique.

Le ciclopirox et ses dérivés sont, en effet, peu solubles dans l'eau.

Les bases moussantes qui ont été étudiées dans le cadre de la présente invention comportent donc un ou plusieurs tensio-actifs dont la fonction est de solubiliser le principe actif et également d'assurer les qualités moussantes de la préparation.

Conformément à la présente invention, la composition pharmaceutique moussante à usage topique destinée au traitement des dermatoses induites par Pityrosporum ovale, est caractérisée en ce qu'elle contient :
(i) à titre de principe actif anti-fongique 0,1 à 10,0% en poids, de préférence 2,1 à 6,0%, de ciclopirox ou de ciclopiroxolamine,
(ii) 0,5% à 35,0% en poids, de préférence de 3 à 20%, et plus particulièrement de 5 à 15%, d'un tensio-actif choisi parmi les alkyl-bétaïnes, les alkyl-amidobétaïnes, les cocamidoalkylamines et leurs dérivés, ainsi que leurs mélanges; ledit tensio-actif constituant le seul solvant du composé (i).

Selon une autre caractéristique particulière de l'invention, la composition est caractérisée en ce que le tensio-actif est une cocamidopropyl bétaïne et/ou le cocamidopropylamine oxyde.

Selon la présente invention, la composition est caractérisée en ce qu'elle se présente sous la forme d'un gel moussant contenant en outre 0,1% à 25%, de préférence 1 à 10 %, d'un agent épaississant et 30% à 99,3% en poids, de préférence 64% à 93,4% et plus particulièrement 69% à 91,9% d'eau.

Selon une autre caractéristique de la présente invention, la composition est caractérisée en ce que l'agent épaissisant est choisi parmi les dérivés de la cellulose, les polyéthylène-glycols, tels que le PEG 6000, le cétéareth-60 myristyl-glycol, ainsi que leurs mélanges.

Conformément à la présente invention, la composition est caractérisée en ce qu'elle se présente sous la forme d'une solution aqueuse moussante contenant de 55% à 99,4% en poids, de préférence de 74 à 94,9% et plus particulièrement de 79% à 92,9% d'eau.

Selon une autre caractéristique particulière de l'invention, la composition est caractérisée en ce qu'elle répond à la composition suivante :

| | |
|---|---|
| Ciclopiroxolamine | 4,0 % |
| Cocamidopropyl bétaïne | 8,0 % |
| Cocamidopropylamine oxyde | 1,0 % |
| Cétéareth-60 myristyl glycol | 4,7 % |
| Parfum | 0,40 % |
| Eau purifiée QSP | 100 % P/P |

Selon la présente invention, la composition est caractérisée en ce qu'elle répond à la composition suivante :

| | |
|---|---|
| Ciclopiroxolamine | 4,0 % |
| Cocamidopropyl bétaïne | 8,0 % |
| Cocamidopropylamine oxyde | 1,0 % |
| Parfum | 0,4 % |
| Eau purifiée QSP | 100 % P/P |

Dans le cadre du développement d'un gel moussant à base de ciclopiroxolamine, nous avons fabriqué les préparations témoin à base de ciclopiroxolamine suivantes :

### Préparation témoin n°1

| | |
|---|---|
| Ciclopiroxolamine | 5 % |
| T.E.A. Lauryl Sulfate | 12 % |
| Sodium C14-C16 Olefin sulfonate | 5% |
| Hydroxy-éthyl cellulose | 0,5% |
| Eau QSP | 100% |

### Préparation témoin n°2

| | |
|---|---|
| Ciclopiroxolamine | 5 % |
| Disodium lauramido MEA sulfosuccinate (et) sodium C12-C14 olefin sulfonate | 15% |
| Soyamide D.E.A. | 3% |
| PEG 120 Méthyl glucose dioléate | 2% |
| Eau QSP | 100% |

Des études microbiologiques (voir méthodologie des études en annexe) ont été effectuées pour comparer l'activité de ces 2 formulations sur le Pytirosporum ovale. Nous avons eu la surprise de constater que l'activité de la ciclopiroxolamine était inhibée dans ces 2 compositions.

Les travaux complémentaires effectués ont montré que cette inhibition était uniquement due aux tensio-actifs, les épaississants n'ayant aucun rôle dans cette inhibition.

La plupart des tensio-actifs moussants, classiquement utilisés pour la formulation de gels moussants ou de solutions moussantes, inhibent *in vitro* l'activité de la ciclopiroxolamine sur le *Pityrosporum ovale* (voir tableau n°1 ci-après).

La méthodologie des essais d'évaluation de l'activité in vitro a été la suivante :

Les recommandations de la Pharmacopée Française Xème édition concernant la détermination de l'activité des préparations antiseptiques miscibles à l'eau ont été appliquées dans le cadre de cette étude.

L'activité est ainsi définie par la capacité d'un produit à réduire le nombre de cellules vivantes dans des conditions de contact représentatives du schéma thérapeutique.

Seules quelques adaptations mineures liées aux exigences nutritives du germe-test ont été apportées.

Le mode opératoire a consisté à mettre en suspension les cellules fongiques dans le produit-test de manière à obtenir la concentration finale maximale en produit, soit 90% dans ce type d'essai.

Le contact a été fixé à 5 minutes à une température de 32°C.

L'action du produit test est ensuite bloquée par le retrait du produit (filtration) ou par dilution / neutralisation.

Les cellules survivantes sont alors dénombrées sur plaques de gélose. La différence entre le titre initial des suspensions et le nombre de cellules survivantes permet de déduire le nombre de cellules tuées.

Les résultats sont exprimés en logarithmes. Ainsi, plus la réduction logarithmique des populations (abréviation : log R) est élevée, plus l'activité fongique des préparations testées est importante.

Le micro-organisme d'essai est la souche F2 de *Malassezia furfur* (synonyme : Pityrosporum ovale, Pityrosporum orbiculare...).

Cette souche est d'origine clinique.

**Tableau 1:**

| Inactivation de la ciclopiroxolamine en présence de divers tensio- actifs sur une population > 10⁶ Pityrosporum ovale (abréviation : P.O.) | |
|---|---|
| Tensio actifs (nom CTFA) inhibant l'activité de la CPO | Activité (log de réduction d'une population de P.O.>10⁶⁾ |
| Référence : solution de CPO à 5% | +++ |

| * TENSIO-ACTIFS ANIONIQUES : | |
|---|---|
| Ether sulfates d'alcool gras | - |
| Sulfates d'alcool gras | - |
| Alcanes sulfonates | - |
| Carboxylates d'alcool gras | - |
| Sulfosuccinates | - |
| Esters de l'acide phosphorique | - |

| * TENSIO-ACTIF AMPHOTERE | |
|---|---|
| Cocamidopropyl bétaïne et glycéryl laurate | - |

| * TENSIO-ACTIF NON IONIQUE | |
|---|---|
| Soyamides DEA | |

| | |
|---|---|
| Légende: + + +: diminution de la population de P.ovale > 5 log | |
| -: diminution de la population de P ovale < 3 log | |

De manière surprenante la demanderesse a mis en évidence le maintien de l'activité de la ciclopiroxolamine avec les tensio-actifs du tableau n°2 :

**Tableau n°2 :**

| activité de la ciclopiroxolamine en présence de divers tensio- actifs sur une population > 10⁶ Pityrosporum ovale (abréviation: P.O.) | |
|---|---|
| Tensio actifs préservant l'activité de la CPO (nom CTFA) | ACTIVITE (log de réduction) d'une population de P.O.>10⁶ |
| Référence : solution de CPO à 5% | +++ |
| cocamidopropylamine oxyde | +++ |
| cocamidopropyl bétaïne | +++ |

| | |
|---|---|
| Légende : + + + : diminution de la population de P.ovale > 5 log | |

Diverses formulations ont ainsi été réalisées avec des dérivés du ciclopirox et les tensio-actifs sus-cités:

### Exemple 1

| | |
|---|---|
| Ciclopiroxolamine | 2,1 à 5,0 % |
| Cocamidopropyl bétaïne | 5,0 à 15,0% |
| Colorant | 0,05 à 1,0% |
| Eau purifiée QSP | 100% |

### Exemple 2

| | |
|---|---|
| Ciclopiroxolamine | 2,5 à 6 % |
| Cocamidopropyl bétaïne | 5 à 15 % |
| Cétéareth 60 myristyl glycol | 0,5 à 10 % |
| Parfum | 0,01 à 2 % |
| Eau purifiée QSP | 100 % |

### Exemple 3 : DC115 GM

| | |
|---|---|
| Ciclopiroxolamine | 4,0 % |
| Cocamidopropyl bétaïne | 8,0 % |
| Cocamidopropylamine oxyde | 1,0 % |
| Cétéareth-60 myristyl glycol | 4,7 % |
| Parfum | 0,40 % |
| Eau purifiée QSP | 100 % P/P |

Les formulations qui ont été développées selon ces exemples présentent un indice d'irritation primaire cutanée compatible avec un usage topique ; elles ont été classées faiblement irritantes ou moyennement irritantes. Ces préparations sont stables au cours du temps.

Le tableau n°3 compare, pour exemple, l'activité d'un gel, préparé selon les revendications ci-après, à l'activité de la ciclopiroxolamine sur une souche d'origine hospitalière F2.

**Tableau n°3:**

| activité de la ciclopiroxolamine et de la formule DC 115 GM sur la souche F2. | | | | | | |
|---|---|---|---|---|---|---|
| Concentration en CPO | 0% | 1% | 2% | 3% | 4% | 5% |
| log de réduction (CPO) | - | - | - | + | ++ | +++ |
| log de réduction (DC115GM) | - | - | - | + | ++ | +++ |

| | | | | | | |
|---|---|---|---|---|---|---|
| Légende - :diminution de la population de P.ovale < 3 log | | | | | | |
| +: diminution de la population de P.ovale comprise entre 3 et 4 log | | | | | | |
| + +: diminution de la population de P.ovale comprise entre 4 et 5 log | | | | | | |
| + + +: diminution de la population de P.ovale > 5 log | | | | | | |

Nous avons de plus ainsi mis en évidence qu'il est nécessaire d'avoir une concentration en sels de ciclopirox supérieure à 2,0 % pour avoir une activité in *vitro* (en 5 minutes de contact) intéressante sur le P.O. (supérieure à 3 log de réduction).

Tous les épaississants classiquement utilisés dans la formulation de gels moussants ont été trouvés compatibles en terme d'activité avec la ciclopiroxolamine; néanmoins pour des raisons de compatibilité physicochimique, la présente invention est limitée aux épaississants stables à pH supérieur ou égal à 7 (la présence de CPO dans les formulations entraîne une augmentation du pH qui se situe aux alentours de 7 pour des préparations avec 1 % de CPO et aux alentours de 9 pour les préparations avec 6% de CPO).

## Revendications

1. Composition pharmaceutique moussante à usage topique destinée au traitement des dermatoses induites par Pityrosporum ovale, **caractérisée en ce qu'**elle contient une association :
(i) de 0,1 à 10,0 % en poids, de préférence 2,1 % à 6,0 % de ciclopirox ou de ciclopiroxolamine à titre de principe actif anti-fongique ;
(ii) de 0,5 % à 35,0 % en poids, de préférence de 3% à 20 %, et plus particulièrement de 5% à 15 %, d'un tensio-actif choisi parmi les alkyl-bétaïnes, les alkyl-amidobétaïnes, les cocamidoalkylamines et leurs dérivés, ainsi que leurs mélanges ; ledit tensio-actif constituant le seul solvant du composé (i).

2. Composition selon la revendication 1, **caractérisée en ce que** le tensio-actif est une cocamidopropyl bétaïne et/ou le cocamidopropylamine oxyde.

3. Composition selon l'une des revendications 1 et 2, **caractérisée en ce qu'**elle se présente sous la forme d'un gel moussant contenant en outre 0,1 % à 25 % en poids, de préférence de 1% à 10,0 %, d'un agent épaississant et 30 % à 99,3 % en poids, de préférence 64 % à 93,4 % et plus particulièrement 69 % à 91,9 % d'eau.

4. Composition selon la revendication 3, **caractérisée en ce que** l'agent épaississant est choisi parmi les dérivés de la cellulose, tels que l'éthyl et la méthyl cellulose, les polyéthylène-glycols, tels que le PEG 6000, le Cétéareth-60 myristyl-glycol, ainsi que leurs mélanges.

5. Composition selon l'une des revendications 1 et 2, **caractérisée en ce qu'**elle se présente sous la forme d'une solution aqueuse moussante contenant de 55 % à 99,4 % en poids, de préférence de 74 % à 94,9 % et plus particulièrement de 79 % à 92,9 % d'eau.

6. Composition selon la revendication 3, **caractérisée en ce qu'**elle répond à la composition suivante :
| | |
|---|---|
| Ciclopiroxolamine | 4,0 % |
| Cocamidopropyl bétaïne | 8,0 % |
| Cocamidopropylamine oxyde | 1,0 % |
| Cétéareth-60 myristyl glycol | 4,7 % |
| Parfum | 0,40 % |
| Eau purifiée QSP | 100 % P/P |

7. Composition selon la revendication 5, **caractérisée en ce qu'**elle répond à la composition suivante :
| | |
|---|---|
| Ciclopiroxolamine | 4,0 % |
| Cocamidopropyl betaïne | 8,0 % |
| cocamidopropylamine oxyde | 1,0 % |
| Parfum | 0,4 % |
| Eau purifiée QSP | 100 % P/P |

8. Utilisation d'une association telle que définie selon la revendication 1 pour la fabrication d'une composition pharmaceutique moussante à usage topique destinée au traitement des dermatoses induites par Pityrosporum ovale.

9. Utilisation selon la revendication 8, **caractérisée en ce que** le tensio-actif est une cocamidopropyl bétaïne et/ou le cocamidopropylamine.

## Claims

1. Foaming pharmaceutical composition for topical use, intended for the treatment of dermatoses induced by Pityrosporum ovale, **characterized in that** it contains a combination:
(i) of 0.1 to 10.0% by weight, preferably 2.1% to 6.0% of ciclopirox or of ciclopiroxolamine as antifungal active ingredient;
(ii) of 0.5% to 35.0% by weight, preferably of 3% to 20%, and more particularly of 5% to 15%, of a surfactant chosen from alkylbetaines, alkylamidobetaines, cocamidoalkylamines and derivatives thereof, as well as mixtures thereof, the said surfactant constituting the only solvent for compound (i).

2. Composition according to Claim 1, **characterized in that** the surfactant is a cocamidopropylbetaine and/or cocamidopropylamine oxide.

3. Composition according to either of Claims 1 and 2, **characterized in that** it exists in the form of a foaming gel containing, inter alia, 0.1% to 25% by weight, preferably from 1% to 10.0%, of a thickening agent and 30% to 99.3% by weight, preferably 64% to 93.4%, and more particularly 69% to 91.9% of water.

4. Composition according to Claim 3, **characterized in that** the thickening agent is chosen from cellulose derivatives such as ethyl and methyl cellulose, polyethylene glycols such as PEG 6000, Ceteareth-60 myristyl-glycol, as well as mixtures thereof.

5. Composition according to either of Claims 1 and 2, **characterized in that** it is provided in the form of a foaming aqueous solution containing from 55% to 99.4% by weight, preferably from 74% to 94.9%, and more particularly from 79% to 92.9% of water.

6. Composition according to Claim 3, **characterized in that** it has the following composition:
| | |
|---|---|
| Ciclopiroxolamine | 4.0% |
| Cocamidopropylbetaine | 8.0% |
| Cocamidopropylamine oxide | 1.0% |
| Ceteareth-60 myristyl glycol | 4.7% |
| Perfume | 0.40% |
| Purified water qs | 100% w/w |

7. Composition according to Claim 5, **characterized in that** it has the following composition:
| | |
|---|---|
| Ciclopiroxolamine | 4.0% |
| Cocamidopropylbetaine | 8.0% |
| Cocamidopropylamine oxide | 1.0% |
| Perfume | 0.4% |
| Purified water qs | 100% w/w |

8. Use of a combination as defined according to Claim 1, for the manufacture of a foaming pharmaceutical composition for topical use intended for the treatment of dermatoses induced by Pityrosporum ovale.

9. Use according to Claim 8, **characterized in that** the surfactant is a cocamidopropylbetaine and/or cocamidopropylamine.

## Patentansprüche

1. Schaumbildende pharmazeutische Zusammensetzung zur topischen Verwendung, die zur Behandlung von Dermatosen bestimmt ist, welche durch Pityrosporum ovale induziert werden, **dadurch gekennzeichnet, dass** sie in Kombination enthält:
(i) 0,1 bis 10,0 Gew.-%, vorzugsweise 2,1 bis 6,0 Gew.-%, Ciclopirox oder Ciclopiroxolamin als Antifungus-Wirkstoff;
(ii) 0,5 bis 35,0 Gew.-%, vorzugsweise 3 bis 20 Gew.-% und spezieller 5 bis 15 Gew.-%, eines Tensids, das ausgewählt ist aus Alkylbetainen, Alkylamidobetainen, Cocamidoalkylaminen und deren Derivaten sowie deren Mischungen; wobei das Tensid das einzige Lösungsmittel der Verbindung (i) ausmacht.

2. Zusammensetzung nach Anspruch 1, **dadurch gekennzeichnet, dass** das Tensid ein Cocamidopropylbetain und/oder Cocamidopropylaminoxid ist.

3. Zusammensetzung nach einem der Ansprüche 1 und 2, **dadurch gekennzeichnet, dass** sie in Form eines schäumenden Gels vorliegt, das darüber hinaus 0,1 bis 25 Gew.-%, vorzugsweise 1 bis 10,0 Gew.-% eines Verdickungsmittels und 30 bis 99,3 Gew.-%, vorzugsweise 64 bis 93,4 Gew.-% und spezieller 69 bis 91,9 Gew.-% Wasser enthält.

4. Zusammensetzung nach Anspruch 3, **dadurch gekennzeichnet, dass** das Verdickungsmittel ausgewählt ist aus Cellulose-Derivaten, wie Ethyl- und Methylcellulose, Polyethylenglycolen, wie PEG 6000, Ceteareth-60-Myristylglycol sowie deren Mischungen.

5. Zusammensetzung nach einem der Ansprüche 1 und 2, **dadurch gekennzeichnet, dass** sie in Form einer schäumenden wässrigen Lösung vorliegt, die 55 bis 99,4 Gew.-%, vorzugsweise 74 bis 94,9 Gew.-% und spezieller 79 bis 92,9 Gew.-% Wasser enthält.

6. Zusammensetzung nach Anspruch 3, **dadurch gekennzeichnet, dass** sie der folgenden Zusammensetzung entspricht:
| | |
|---|---|
| Ciclopiroxolamin | 4,0 % |
| Cocamidopropylbetain | 8,0 % |
| Cocamidopropylaminoxid | 1,0 % |
| Ceteareth-60-myristylglycol | 4,7 % |
| Parfüm | 0,40 % |
| gereinigtes Wasser, q.s.p. | 100 % G/G |

7. Zusammensetzung nach Anspruch 5, **dadurch gekennzeichnet, dass** sie der folgenden Zusammensetzung entspricht:
| | |
|---|---|
| Ciclopiroxolamin | 4,0 % |
| Cocamidopropylbetain | 8,0 % |
| Cocamidopropylaminoxid | 1,0 % |
| Parfüm | 0,4 % |
| gereinigtes Wasser, q.s.p. | 100 % G/G |

8. Verwendung einer Kombination, wie gemäß Anspruch 1 definiert, für die Herstellung einer schäumenden pharmazeutischen Zusammensetzung zur topischen Verwendung, die zur Behandlung von Dermatosen bestimmt ist, die durch Pityrosporum ovale induziert werden.

9. Verwendung nach Anspruch 8, **dadurch gekennzeichnet, dass** das Tensid ein Cocamidopropylbetain und/oder Cocamidopropylaminoxid ist.
